# EUROPEAN PATENT SPECIFICATION

(11) **EP 0 837 672 B1**
(45) Date of publication and mention of the grant of the patent: **19.11.2003**
(21) Application number: 96924371.6
(22) Date of filing: 02.07.1996
(51) Int. Cl.: A61K 9/127, A61K 38/00, A61K 45/00, A61K 47/00, A61K 47/44, A01N 37/18, A61K 9/00, A61K 38/17, A61K 39/00

(54) **PREPARATIONS AND METHODS FOR THE TREATMENT OF T CELL MEDIATED DISEASES**
MITTEL UND VERFAHREN ZUR BEHANDLUNG T-ZELLEN VERMITTELTER KRANKHEITEN
PREPARATIONS ET PROCEDES DE TRAITMENTS DE MALADIES MEDIEES PAR LES LYMPHOCYTES T

(30) Priority: 05.07.1995 IL 11445895
(43) Date of publication of application: 29.04.1998
(73) Proprietor: YEDA RESEARCH AND DEVELOPMENT CO. LTD., Rehovot 76100 (IL)
(72) Inventor: COHEN, Irun, R., 76344 Rehovot (IL); ELIAS, Dana, 70 700 Gedera (IL); SHINITZKY, Meir, 46910 Kfar Shmaryahu (IL)
(74) Representative: Lönnqvist, Gunnel Solveig Kristina
(86) International application number: US9611373
(87) International publication number: WO97002016

(56) References cited:
- WO-A-91/15225
- US-A- 4 395 394
- US-A- 4 474 773
- US-A- 5 114 844
- US-A- 5 254 339
- CELL, 20 October 1989, Vol. 59, URBAN et al., "Autoimmune T Cells: Immune Recognition of Normal and Variant Peptide Epitopes and Peptide-Based Therapy", pages 257-271.
- SPRINGER SEMINARS IN IMMUNOPATHOLOGY, 1992, Vol. 14, WRAITH et al., "A Role for Major Histocompatibility Complex-Binding Peptides in the Immunotherapy of Autoimmune Disease", pages 95-101.
- PROC. NATL. ACAD. SCI. U.S.A., April 1991, Vol. 88, ELIAS et al., "Vaccination Against Autoimmune Mouse Diabetes with a T-cell Epitope of the Human 65-kDa Heat Shock Protein", pages 3088-3091.
- SPRINGER SEMIN. IMMUNOPATHOL., 1996, Vol. 18, MARTIN et al., "Experimental Immunotherapies for Multiple Sclerosis", pages 1-24.

### SEQUENCE LISTING

(1) GENERAL INFORMATION:
   (i) APPLICANT:
      (A) NAME: YEDA RESEARCH & DEVELOPMENT CO. LTD. at the weizmann Institute of Science
      (B) STREET: P.O. Box 95
      (C) CITY: Rehovot
      (E) COUNTRY: Israel
      (F) POSTAL CODE (ZIP): 76100
      (G) TELEPHONE: 972-8-470739
      (H) TELEFAX: 972-8-4706178

      (A) NAME: Irun R. COHEN
      (B) STREET: 11 Hankin Street
      (C) CITY: Rehovot
      (E) COUNTRY: Israel
      (F) POSTAL CODE (ZIP) : 76344

      (A) NAME: Dana ELIAS
      (B) STREET: 57 Derech Yavne
      (C) CITY: Rehovot
      (E) COUNTRY: Israel
      (F) POSTAL CODE (ZIP): 76344

      (A) NAME: Meir SHINITZKY
      (B) STREET: 20 Derech Haganim
      (C) CITY: Kfar Shmaryahu
      (E) COUNTRY: Israel
      (F) POSTAL CODE (ZIP): 46910
   (ii) TITLE OF INVENTION: PREPARATIONS AND METHODS FOR THE TREATMENT OF T CELL MEDIATED DISEASES
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: IL 114458
      (B) FILING DATE: 05-JUL-1995
(2) INFORMATION FOR SEQ ID NO: 1:
   (i) SEQUENCE CHARACTERISTICS:
      (A) LENGTH: 573 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 1:
   Met Leu Arg Leu Pro Thr Val Phe Arg Gln Met Arg Pro Val Ser Arg
   1               5                   10                  15
   Val Leu Ala Pro His Leu Thr Arg Ala Tyr Ala Lys Asp Val Lys Phe
               20                  25                  30
   Gly Ala Asp Ala Arg Ala Leu Met Leu Gln Gly Val Asp Leu Leu Ala
           35                  40                  45
   Asp Ala Val Ala Val Thr Met Gly Pro Lys Gly Arg Thr Val Ile Ile
       50                  55                  60
   Glu Gln Gly Trp Gly Ser Pro Lys Val Thr Lys Asp Gly Val Thr Val
   65                  70                  75                  80
   Ala Lys Ser Ile Asp Leu Lys Asp Lys Tyr Lys Asn Ile Gly Ala Lys
                   85                  90                  95
   Leu Val Gln Asp Val Ala Asn Asn Thr Asn Glu Glu Ala Gly Asp Gly
               100                 105                 110
   Thr Thr Thr Ala Thr Val Leu Ala Arg Ser Ile Ala Lys Glu Gly Phe
           115                 120                 125
   Glu Lys Ile Ser Lys Gly Ala Asn Pro Val Glu Ile Arg Arg Gly Val
       130                 135                 140
   Met Leu Ala Val Asp Ala Val Ile Ala Glu Leu Lys Lys Gln Ser Lys
   145                 150                 155                 160
   Pro Val Thr Thr Pro Glu Glu Ile Ala Gln Val Ala Thr Ile Ser Ala
                   165                 170                 175
   Asn Gly Asp Lys Glu Ile Gly Asn Ile Ile Ser Asp Ala Met Lys Lys
               180                 185                 190
   Val Gly Arg Lys Gly Val Ile Thr Val Lys Asp Gly Lys Thr Leu Asn
           195                 200                 205
   Asp Glu Leu Glu Ile Ile Glu Gly Met Lys Phe Asp Arg Gly Tyr Ile
       210                 215                 220
   Ser Pro Tyr Phe Ile Asn Thr Ser Lys Gly Gln Lys Cys Glu Phe Gln
   225                 230                 235                 240
   Asp Ala Tyr Val Leu Leu Ser Glu Lys Lys Ile Ser Ser Ile Gln Ser
                   245                 250                 255
   Ile Val Pro Ala Leu Glu Ile Ala Asn Ala His Arg Lys Pro Leu Val
               260                 265                 270
   Ile Ile Ala Glu Asp Val Asp Gly Glu Ala Leu Ser Thr Leu Val Leu
           275                 280                 285
   Asn Arg Leu Lys Val Gly Leu Gln Val Val Ala Val Lys Ala Pro Gly
       290                 295                 300
   Phe Gly Asp Asn Arg Lys Asn Gln Leu Lys Asp Met Ala Ile Ala Thr
   305                 310                 315                 320
   Gly Gly Ala Val Phe Gly Glu Glu Gly Leu Thr Leu Asn Leu Glu Asp
                   325                 330                 335
   Val Gln Pro His Asp Leu Gly Lys Val Gly Glu Val Ile Val Thr Lys
               340                 345                 350
   Asp Asp Ala Met Leu Leu Lys Gly Lys Gly Asp Lys Ala Gln Ile Glu
           355                 360                 365
   Lys Arg Ile Gln Glu Ile Ile Glu Gln Leu Asp Val Thr Thr Ser Glu
       370                 375                 380
   Tyr Glu Lys Glu Lys Leu Asn Glu Arg Leu Ala Lys Leu Ser Asp Gly
   385                 390                 395                 400
   Val Ala Val Leu Lys Val Gly Gly Thr Ser Asp Val Glu Val Asn Glu
                   405                 410                 415
   Lys Lys Asp Arg Val Thr Asp Ala Leu Asn Ala Thr Arg Ala Ala Val
               420                 425                 430
   Glu Glu Gly Ile Val Leu Gly Gly Gly Cys Ala Leu Leu Arg Cys Ile
           435                 440                 445
   Pro Ala Leu Asp Ser Leu Thr Pro Ala Asn Glu Asp Gln Lys Ile Gly
       450                 455                 460
   Ile Glu Ile Ile Lys Arg Thr Leu Lys Ile Pro Ala Met Thr Ile Ala
   465                 470                 475                 480
   Lys Asn Ala Gly Val Glu Gly Ser Leu Ile Val Glu Lys Ile Met Gln
                   485                 490                 495
   Ser Ser Ser Glu Val Gly Tyr Asp Ala Met Ala Gly Asp Phe Val Asn
               500                 505                 510
   Met Val Glu Lys Gly Ile Ile Asp Pro Thr Lys Val Val Arg Thr Ala
           515                 520                 525
   Leu Leu Asp Ala Ala Gly Val Ala Ser Leu Leu Thr Thr Ala Glu Val
       530                 535                 540
   Val Val Thr Glu Ile Pro Lys Glu Glu Lys Asp Pro Gly Met Gly Ala
   545                 550                 555                 560
   Met Gly Gly Met Gly Gly Gly Met Gly Gly Gly Met Phe
                   565                 570
(2) INFORMATION FOR SEQ ID NO: 2:
   (i) SEQUENCE CHARACTERISTICS:
      (A) LENGTH: 24 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 2:
   Val Leu Gly Gly Gly Val Ala Leu Leu Arg Cys Ile Pro Ala Leu Asp
   1               5                   10                  15
   Ser Leu Thr Pro Ala Asn Glu Asp
               20
(2) INFORMATION FOR SEQ ID NO: 3:
   (i) SEQUENCE CHARACTERISTICS:
      (A) LENGTH: 24 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 3:
   Val Leu Gly Gly Gly Cys Ala Leu Leu Arg Val Ile Pro Ala Leu Asp
   1               5                   10                  15
   Ser Leu Thr Pro Ala Asn Glu Asp
               20
(2) INFORMATION FOR SEQ ID NO: 4:
   (i) SEQUENCE CHARACTERISTICS:
      (A) LENGTH: 24 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 4:
   Val Leu Gly Gly Gly Val Ala Leu Leu Arg Val Ile Pro Ala Leu Asp
   1               5                   10                  15
   Ser Leu Thr Pro Ala Asn Glu Asp
               20

## Description

### Field of the Invention

The present invention relates to vaccine therapy for T-cell mediated autoimmune diseases, and in particular to therapeutic preparations comprising antigens recognized by T cells involved in the pathogenesis of T cell mediated autoimmune diseases, and a metabolizable lipid emulsion as a biologically active carrier.

### Background of the Invention

Autoimmune disorders, e.g., insulin-dependent diabetes mellitus (IDDM or type I diabetes), multiple schlerosis, rheumatoid arthritis and thyroiditis, are characterized by reactivity of the immune system to an endogenous antigen, with consequent injury to tissues. These immune responses to self-antigens are maintained by the persistent activation of self-reactive T lymphocytes.

T cells of the CD4 "helper" type have been divided into two groups by the characteristic cytokines they secrete when activated (Mosmann and Coffman, 1989). TH1 cells secrete IL-2, which induces T cell proliferation, and cytokines such as IFN-γ, which mediate tissue inflammation. TH2 cells, in contrast, secrete IL-4 and IL-10. IL-4 helps T cells secrete antibodies of certain IgG isotypes and suppresses the production of TH1 inflammatory cytokines (Banchereau et al., 1994). IL-10 indirectly inhibits TH1 activation by affecting antigen-presentation and inflammatory cytokine production by macrophages (Moore et al., 1993). It is the TH1 cells which contribute to the pathogenesis of organ-specific autoimmune diseases. TH1-type responses also appear to be involved in other T cell mediated diseases or conditions, such as contact dermatitis (Romagnani, 1994).

Peptides suitable for immunologically specific therapy of an autoimmune disease are peptides that are recognized by T cells involved in the pathogenesis of the autoimmune disease. Each autoimmune disease will have its ideal peptide for use in therapy. A disease like multiple sclerosis involving T cells reactive to self-antigens such as myelin basic protein (MBP) (Allegreta et al., 1990) will require a peptide of myelin basic protein for its therapy, as for example those described by Ota et al., 1990.

The present inventors have shown that autoimmune diseases such as type I diabetes mellitus may be treated by administering a suitable peptide in an oil vehicle. NOD mice spontaneously develop type I diabetes caused by autoimmune T cells that attack the insulin-producing β cells of the islets. The autoimmune attack is associated with T-cell reactivity to a variety of self-antigens including a peptide of the 60kDa heat shock protein (hsp 60) and peptides of glutamic acid decarboxylase (GAD). Thus, for example, spontaneous diabetes developing in the NOD/Lt strain of mice could be treated with a peptide designated p277 corresponding to positions 437-460 of. the human hsp 60 sequence (PCT Patent Publication No. WO90/10449; D. Elias and I.R. Cohen, Peptide therapy for diabetes in NOD mice, The Lancet 343:704-06, 1994); with variants of the p277 peptide in which one or both cysteine residues at positions 6 and/or 11 have been replaced by valine and/or the Thr residue at position 16 is replaced by Lys (see PCT Publication WO96/19236) and with peptides designated p12 and p32 corresponding to positions 166-185 and 466-485, respectively, of the human hsp60 sequence. See Israel Patent Application No. 114,407 of the same applicant of the present application, filed on June 30, 1995. See also International Patent Application WO 97/01959 filed July 1, 1996, claiming priority from said Israel application no. 114,407.

Peptide therapy for treatment of IDDM using p12, p32, p277 or variants thereof, was found by the present inventors to be effective when the peptide was administered to mice subcutaneously (sc) in an oil vehicle such as an emulsion of mineral oil known as incomplete Freund's adjuvant (IFA). However, IFA as well as complete Freund's adjuvant (CFA; a preparation of mineral oil containing various amounts of killed organisms of Mycobacterium) are not allowed for human use because the mineral oil is not metabolizable and cannot be degraded in the body. Therefore, it would be desirable to discover an effective vehicle for peptide therapy that would be metabolizable.

Several fat emulsions have been in use for many years for intravenous nutrition of human patients. Two of the available commercial fat emulsions, known as Intralipid ("Intralipid" is a registered trade mark of Kabi Pharmacia, Sweden, for a fat emulsion for intravenous nutrition, described in US Patent No. 3,169,094) and Lipofundin (a registered trade mark of B. Braun Melsungen, Germany) contain soybean oil as fat (100 or 200 g in 1,000 ml distilled water: 10% or 20%, respectively). Egg-yolk phospholipids are used as emulsifiers in Intralipid (12g/l distilled water) and egg-yolk lecithin in Lipofundin (12g/l distilled water). Isotonicity results from the addition of glycerol (25g/l) both in Intralipid and in Lipofundin. These fat emulsions are quite stable and have been used for intravenous nutrition of patients suffering from gastrointestinal or neurological disorders, which prevent them from receiving nutrition orally, and thus they receive the calories needed to sustain life. Usual daily doses are of up to 1 liter daily.

US Patents No. 4,073,943 issued on February 14, 1978 to Wretlind et al. and Re. 32,393 issued on May 29, 1990 as reissue patent of US No. 4,168,308 issued on September 18, 1979 to Wretlind et al., describe a carrier system for use in enhancing parenteral, particularly intravenous, administration of a pharmacologically active, oil-soluble agent, comprising a stable, oil-in-water emulsion containing a pharmacologically inert lipoid as a hydrophobic phase dispersed in a hydrophilic phase, said lipid being dispersed in the emulsion as finely divided particles having a mean particle size less than 1 micron to achieve rapid onset of an acceptable therapeutic effect, said carrier system being used with an effective dose of said pharmacologically active, oil-soluble agent predominantly dissolved in said lipoid at a fraction ratio thereto in the hydrophobic phase, said therapeutic effect being attributable to said effective dose of the active agent. This carrier system is said to be suitable for administration of a water-insoluble or water-soluble, oil-soluble pharmacologically active agent that is predominantly dissolved in the lipoid phase. Examples of such pharmacologically active agents are depressants, anaesthetics, analgesics, stimulants, spasmolytics, muscle relaxants, vasodepressants and diagnostic, e.g. X-ray contrast, agents. The carrier system is said to enhance the diagnostic or therapeutic effect of the agent with a rapid onset accompanied by a reduced incidence of injury to body tissues.

US-A-5,254,339 discloses for improving immunity the use of external solubilized antigens formulated as specific complexes with critical micellar concentrations and glycosides:
US-A-4,474,773 discloses an active lipid fraction from natural sources, which is used for the treatment e.g. of self-caused dysfunction of the immune system and drug withdrawal symptoms. Neither US-A-5,254,339 or US-A4,474,773 discloses emulsion. US-A-4,395,394 discloses'a vaccine adjuvant comprising external antigens including pathogenic organisms, vira and allergens in combination with a novel lipid amine component. None of the above US-patents suggests the use of an emulsion as a carrier for an antigen in the treatment of T-cell mediated diseases.

Intralipid has been proposed as a non-irritating vehicle for several adjuvants for use in vaccines such as, for example, 6-O-(2-tetradecylhexadecanoyl)- and 6-O-(3-hydroxy-2-docosylhexacosanoyl)-N-acetylmuramyl-L-alanyl-D-isoglutamine (Tsujimoto et al., 1986 and 1989), avridine (Woodard and Jasman, 1985), N,N-dioctadecyl-N',N'-bis(2-hydroxyethyl) propanediamine (CP-20,961) (German Patent Application No. DE 2945788; Anderson and Reynolds, 1979; Niblack et al., 1979). Kristiansen and Sparrman, 1983, have disclosed that the immunogenicity of hemagglutinin and neuraminidase.in mice is markedly increased after adsorption onto lipid particles constituting Intralipid.

None of the above publications describe the use of Intralipid as a vehicle for peptides in the treatment of . autoimmune diseases, nor has there been any disclosure that Intralipid could mediate a shift of the immune response from a TH1-type response to a TH2-type response.

### Summary of the Invention

It has now been found, in accordance with the present invention, that metabolizable lipid emulsions, such as Intralipid and Lipofundin, can act as vehicles for peptide therapy of T cell mediated autoimmune diseases or conditions. It has been further found that this activity is associated with a TH1 to TH2 cytokine shift.

The present invention thus relates to a therapeutic preparation for the treatment of a T cell mediated autoimmune disease or condition, comprising a peptide or other antigen and a biologically active lipid carrier, wherein the peptide or other antigen is one recognized by inflammatory T-cells associated with the pathogenesis of said disease or condition, and wherein the biologically active lipid carrier is a fat emulsion comprising 10-20% triglycerides of plant and/or animal origin, 1.2-2.4% phospholipids of plant and/or animal origin, 2.25-4.5% osmo-regulator, 0-0.05% anti-oxidant, and sterile water to 100%.

The triglycerides and phospholipids of plant or animal origin may derive from any suitable vegetable oil, such as soybean oil, cottonseed oil, coconut oil or olive oil, or from egg-yolk or bovine serum. Preferably, the triglycerides are derived from soybean oil and the phospholipids are derived from soybean or from egg-yolk. Preferably, the triglycerides/phospholipids weight ratio is about 8:1.

Any suitable osmo-regulator may be added to the fat emulsion, preferably glycerol, xylitol or sorbitol. The fat emulsion may optionally comprise an anti-oxidant, for example 0.05% tocopherol.

In one embodiment of the invention, the fat emulsion as defined above is processed by centrifugation, e.g. at 10,000g or higher, thus forming a small triglyceride-rich (about 90% triglycerides) layer on the top of a phospholipid-enriched aqueous dispersion containing about 1:1 triglycerides:phospholipids, and this latter aqueous dispersion is used as the lipid vehicle in the preparations of the invention.

In one preferred embodiment of the invention, the preparation is for the treatment of insulin-dependent diabetes mellitus (IDDM) and comprises a peptide derived from the human heat shock protein 60 (hsp60) that is recognized by inflammatory T-cells associated with the pathogenesis of IDDM, wherein said peptide is selected from the group of peptides appearing in the following Table 1:

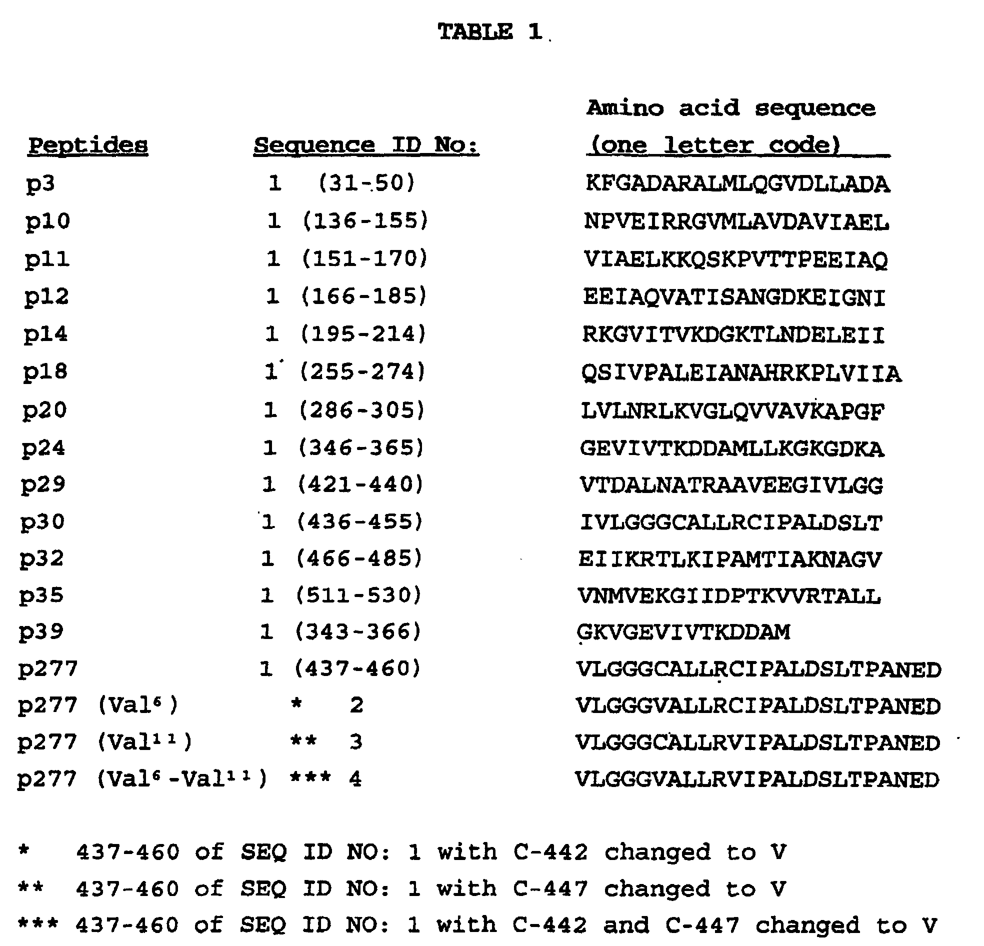

The invention further relates to a method for therapy of a subject suffering from an autoimmune disease or other TH1 mediated disease or condition, which comprises administering to said subject an effective amount of a therapeutic preparation according to the invention.

### Brief Description of the Drawings

Fig. 1 shows anti-p277 antibody production in NOD mice treated with the peptide p277(Val⁶-Val¹¹) in: (i) Intralipid or (ii) phosphate-buffered saline (PBS), as described in Example 2.
Fig. 2 shows TH2-dependent antibody isotypes induced in NOD mice by treatment with the peptide p277(Val⁶-Val¹¹) in Intralipid, as described in Example 3.

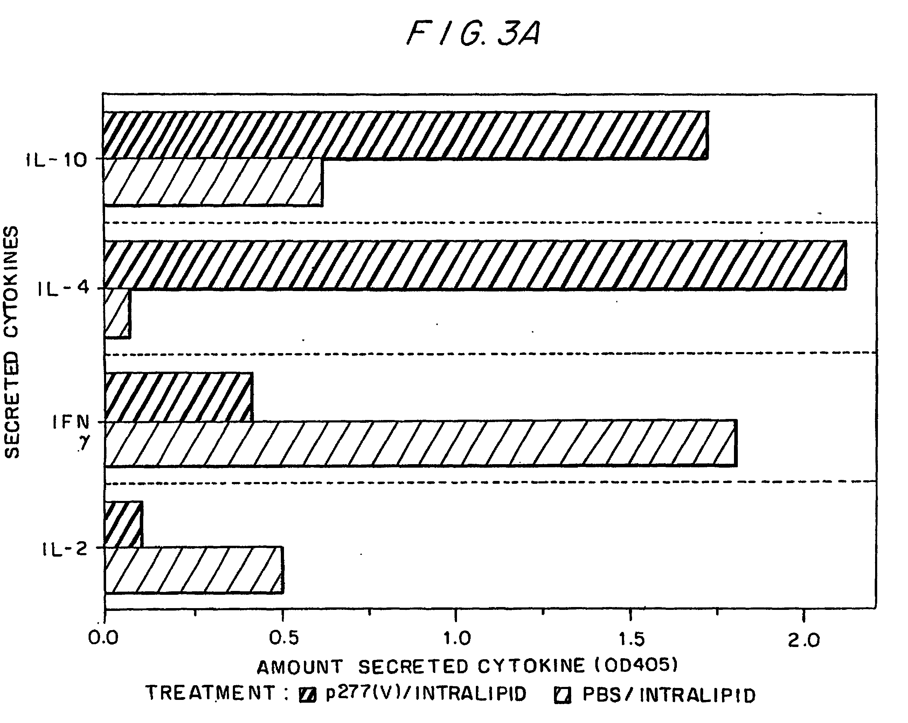

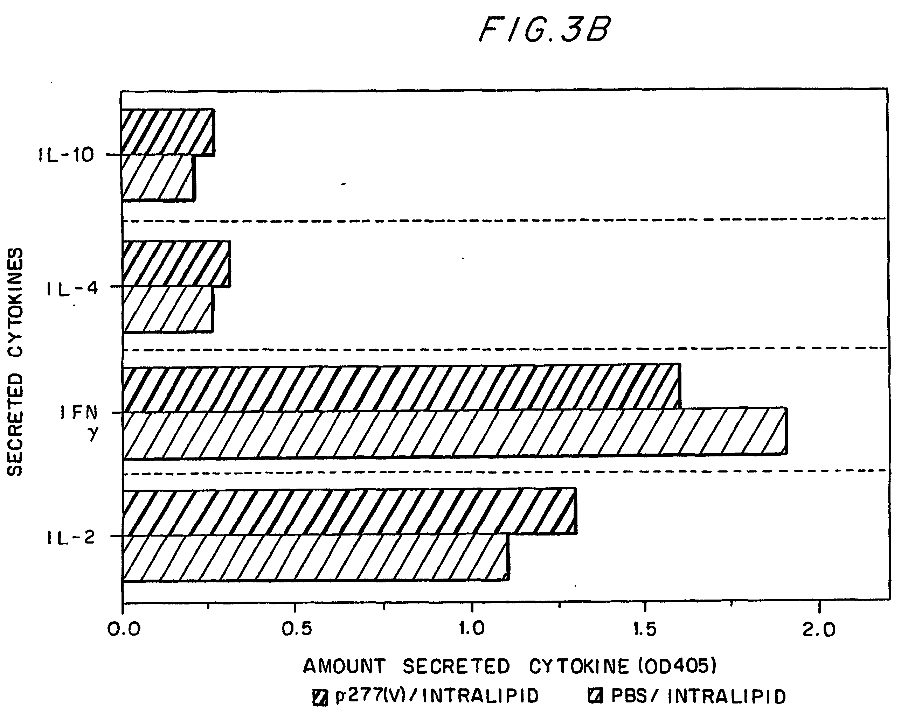

Figs. 3A-B show that p277(Val⁶-Val¹¹)/ Intralipid therapy induces in NOD mice a specific switch in the profile of cytokines produced by the T-cells reactive to the p277(Val⁶-Val¹¹) peptide, as described in Example 4. Fig. 3A shows that there is a reduction of TH1 (IL-2, IFN-γ) and elevation of TH2 (IL-4, IL-10) cytokines after treatment of the mice with the p277(Val⁶-Val¹¹) peptide in Intralipid and incubation of the spleen cells with p277(Val⁶-Val¹¹); Fig. 3B shows that there is no change in the cytokines after treatment of the mice with the p277(Val⁶-Val¹¹) peptide in Intralipid and incubation of the spleen cells with Con A.
Fig. 4 shows that spontaneous T-cell proliferative responses to p277(Val⁶-Val¹¹) is reduced after treatment with the p277(Val⁶-Val¹¹) peptide in Intralipid, as described in Example 5.
Fig. 5 shows that treatment of rats with myelin basic protein peptide p71-90 in Intralipid reduces the severity of experimental autoimmune encephalomyelitis (EAE), as described in Example 6.
Fig. 6 shows that treatment of rats with myelin basic protein peptide p71-90 in IFA reduces the severity of experimental autoimmune encephalomyelitis (EAE), as described in Example 6.

### Detailed Description of the Invention

According to the present invention, it was found that p277(Val⁶-Val¹¹)-peptide treatment, in an appropriate carrier, down-regulated the spontaneous T-cell proliferative responses to epitopes of both hsp60 and GAD and abolished the production of autoantibodies to hsp60, to GAD and to insulin. Arrest of the disease process was associated, not with T-cell tolerance or anergy, but with a shift in the cytokines produced by the autoimmune T cells reactive to p277(Val⁶-Val¹¹) from a TH1-like profile (IL-2, IFNγ) to a TH2-like profile (IL-4, IL-10). The modulation was immunologically specific; the spontaneous T-cell response of the treated mice to a bacterial hsp6O peptide remained in the TH1 mode. Thus, the diabetogenic process characterized by autoimmunity to several self antigens can be cured using one of the antigens, e.g., peptide p277(Val⁶-Val¹¹).

The association of p277(Val⁶-Val¹¹) therapy with a switch in reactivity to p277(Val⁶-Val¹¹) from T-cell proliferation to antibodies indicates that the therapeutic effect results from a shift in the predominant cytokines produced by the autoimmune T cells in the treated mice. TH1 cells secrete IL-2, which induces T-cell proliferation, and cytokines such as IFN-γ, which mediate tissue inflammation, thereby contributing to the pathogenesis of the disease; TH2 cells, in contrast, secrete IL-4 and IL-10. IL-4 helps B cells secrete antibodies of certain IgG isotypes and suppresses the production of TH1 inflammatory cytokines. IL-10 indirectly inhibits TH1 activation by affecting antigen-presentation and inflammatory cytokine production by macrophages. Thus, TH2 cells suppress TH1 activity (see Liblau et al., 1995). The shift from TH1 to TH2-like behavior was supported. by analysis of the isotypes of the antibodies produced before and after p277(val⁶-Val¹¹) therapy.

The fact that the mechanism of the therapeutic effect of the peptide in a lipid vehicle treatment is shown to involve a TH1→TH2 cytokine shift, provides the possibility of using the TH1→TH2 shift as evidence that the treatment was effective and did induce a beneficial response. In other words, the TH1→TH2 shift can serve as a surrogate marker of the response to treatment. For example, the lack of the shift can indicate a need for a second treatment. See Israel Patent Application No. 114,459 filed on July 5, 1995.

The lipid emulsions of the present invention, when used as a vaccine adjuvant with the antigenic substance to which the T cells involved in the disease or condition being treated are active, serve to mediate a shift from a TH1 T cell response prior to treatment to a TH2 T cell response after treatment. This finding establishes that such lipid emulsions are tolerogenic biologically active carriers which can be used in vaccines for the treatment of any TH1 mediated disease or condition. In such vaccines, the antigen provides the immunological specificity for a therapeutic effect while the biologically active carrier of the present invention provides the biological outcome, i.e., the TH1→TH2 shift. Because of the shift mediated by said biologically active carrier of the present invention, diseases with a spectrum of autoreactivities can be turned off with a single antigen/carrier combination capable of inducing a T cell cytokine shift.

A preferred use in accordance with the present invention is in the treatment of organ-specific autoimmune diseases which are mediated by TH1 cells. Such diseases include autoimmune diseases such as IDDM, rheumatoid arthritis, multiple sclerosis and thyroiditis. The peptide used in such treatment is an autoantigen peptide. Thus, for example, for IDDM the peptide is the above-mentioned p277 peptide or the valine substituted analog p277(Val⁶-Val¹¹); for multiple sclerosis such peptide is derived from myelin basic protein; for thyroiditis the peptide is thought to be derived from thyroglobulin, and for rheumatoid arthritis the autoantigen can derive from mycobacterium organisms, e.g., *Mycobacterium tuberculosis*.

It is not critical that the antigen be a peptide. Thus, for example, TH1-mediated allergic responses which result in skin sensitivity and inflammation, such as contact dermatitis, can be treated by a vaccine containing the irrititant antigen and a biologically active carrier in accordance with the present invention which will cause a shift in the cytokine response from a TH1-type to a TH2-type. Thus, while the patient will continue to have elevated antibody levels against the antigen, the inflammatory T cell response causing the skin irritation will be suppressed.

Accordingly, the tolerogenic biologically active carrier of the present invention may be used any time that it is desired to create tolerance for the antigen which the T cells are attacking, i.e., any time that a vaccine is being used to restrict a T cell mediated condition, particularly a TH1 cell mediated condition. If it can be determined which antigen is activating the response in graft rejection or in graft-versus-host disease, then the administration of such an antigen with a carrier in accordance with the present invention would be expected to facilitate the shift of the undesirable inflammatory TH1 response to a more desirable TH2 response, regardless of the overall complexity of the number of antigens to which T cells are active in such condition.

To determine the T-cell secretion of cytokines following activation with peptides, lymphocytes from the peripheral blood of patients are tested in an *in vitro* activation assay. Peripheral blood lymphocytes are isolated from whole heparinized blood on ficol-hypaque, and cultured with the test peptide(s) at concentrations of 5-50 pg/ml. The supernatants from the cultured T-cells are collected at different time points and tested for activity of various cytokines, by ELISA or bioassay(s).

Examples of fat emulsions that can be used in the preparations of the present invention include the commercially available Intralipid and Lipofundin for intravenous nutrition, and the fat emulsions described in the above-mentioned US Patents Nos. 3,169,096, 4,073,943 and 4,168,308. However, the finding according to the present invention that these metabolizable lipids, administered previously for intravenous nutrition, may be used effectively as vehicles for therapy of T cell mediated diseases, is completely unexpected. Similarly, the discovery that these preparations are tolerogenic biologically active carriers which mediate a TH1→TH2 shift is also totally unexpected.

The fat emulsions of the present invention are preferably used as freshly prepared or after storage in a container which is not open to the atmospheric air. Prolonged storage of Intralipid, for example, while exposed to atmospheric air, causes a decrease in the pH and a corresponding decrease in the biological activity.

In one embodiment, the biologically active carrier of the invention is a fat emulsion comprising 10% soybean oil, 1.2% egg-yolk phospholipids, 2.5% glycerol and sterile water to complete 100 % (Intralipid 10%). In another embodiment, the vehicle is a fat emulsion comprising 20% soybean oil, 2.4% egg-yolk phospholipids, 2.5% glycerol and sterile water to complete 100 %.

In yet another embodiment, the vehicle is a fat emulsion comprising 5% soybean oil and another 5% triglycerides from animal origin, e.g. 5% medium chain triglycerides from butter, 1.2% egg-yolk lecithin, 2.5% glycerol and distilled water to complete 100 % (Lipofundin 10%).

In one embodiment of the invention, the vehicle is a processed lipid emulsion obtained by centrifugation, e.g. at 10,000g or higher, of the original fat emulsion defined herein, whereby a small triglyceride-rich (about 90% triglycerides) is formed on the top of a phospholipid-enriched aqueous dispersion containing about 1:1 triglycerides:phospholipids. The two phases are separated and the phospholipid-rich aqueous dispersion is used as the vehicle.

The preparations of the invention may comprise one or more peptides. Thus, for example, for the treatment of IDDM, the preparation may comprise one or more of the peptides p12, p32, p277, p277(Val⁶), p277(Val¹¹), p277(Val⁶ - Val¹¹), or any of the other peptides of Table 1. In one preferred embodiment, the preparation for the treatment of IDDM comprises a peptide p277 or p277(val⁶-Val¹¹) and a fat emulsion comprising 10% soybean oil, 1.2% egg-yolk phospholipids, 2.5% glycerol and sterile water to complete 100 % (Intralipid 10%).

The invention further relates to the use of a fat emulsion as defined herein or of a processed phospholipid-enriched aqueous dispersion prepared therefrom by centrifugation for the preparation of a therapeutic preparation comprising one or more peptides or other'antigens and said fat emulsion or processed aqueous dispersion as a vehicle in the therapy of T cell mediated autoimmune diseases or conditions.

The invention will now be illustrated by the examples.

### EXAMPLES

### Example 1.

### Peptide therapy of type I diabetes using p277(Val⁶-Val¹¹) in oils

The efficacy of various lipid preparations as vehicles for peptide therapy of the diabetes of NOD mice was tested. In this model, autoimmune destruction of the insulin producing β-cells in the pancreas is mediated by T-lymphocytes. An inflammatory infiltrate develops around the pancreatic islets at 5-8 weeks of age and β-cell destruction leading to insulin deficiency and overt diabetes becomes manifested at 14-20 weeks of age affecting almost 100% of female NOD mice by 35-40 weeks of age.

NOD female mice were treated with 100 µg of peptide p277(Val⁶-Val¹¹) per mouse sc in 0.1 ml of: (i) Phosphate-buffered saline (PBS), or (ii) a 10% lipid emulsion composed of 10% soybean oil, 1.2% egg phospholipids and 2.25% glycerol (Intralipid, Kabi Pharmacia AB, Sweden).

The incidence of diabetes at 6 months of age and the production of anti-p277(Val⁶-Val¹¹) antibodies was followed. Diabetes was diagnosed as persistent hyperglycemia, blood glucose levels over 11 mmol/L measured at least twice at weekly intervals with a Beckman Glucose Analyzer II. Successful peptide treatment was assayed by maintenance of a normal blood glucose concentration (less than 11 mmol/L), remission of the intra-islet inflammation of the pancreatic islets (insulitis) and induction of antibodies to the therapeutic peptide as an indicator of a TH2-type immune response. The results are shown in Table 2.

**Table 2:**

| **Incidence of Diabetes at 6 months.** | | |
|---|---|---|
| **Treatment (%)** | **Diabetes** | **Death (%) incidence** |
| p277(Val²-Val¹¹)/PBS | 90 | 80 |
| p277(Val⁶-Val¹¹)/Intralipid | 45# | 20# |
| none | 100 | 90 |
| # p<0.01 compared to untreated NOD mice. | | |

As can be seen from Table 2, peptide treatment administered in Intralipid was effective in reducing the incidence of diabetes and death. On the other hand, treatment administered in PBS was ineffective.

### Example 2.

### Anti-p277(Val⁶-Val¹¹) antibody production

The protection from diabetes by treatment with the p277(Val⁶-Val¹¹) peptide is dependent on TH2 immunological reactivity to the peptide. Therefore, antibody production was measured in the p277(Val⁶-Val¹¹)-immunized mice by ELISA. Maxisorp microtiter plates (Nunc) were coated with p277(Val⁶-Val¹¹) peptide, 10 µg/ml, for 18h and non-specific binding blocked with 7% milk powder for 2h. The mouse sera, diluted 1:50, were allowed to bind for 2h and the specific binding was detected by adding alkaline phosphatase anti-mouse IgG (Serotec) for 2h and p-nitrophenylphosphate substrate (Sigma) for 30 min. The color intensity was measured by an ELISA reader (Anthos) at OD=405 nm.

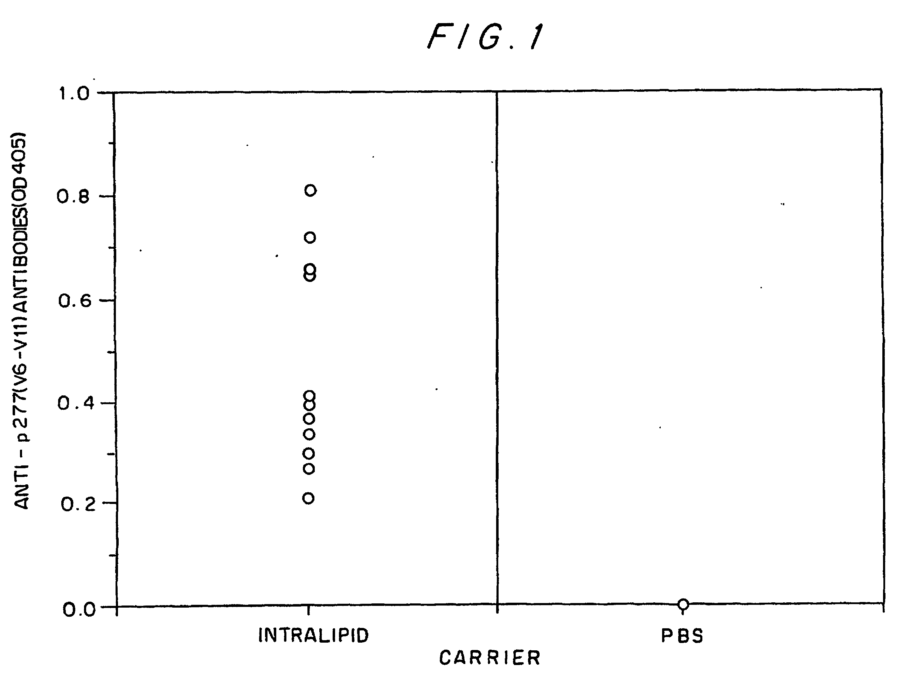

As can be seen from Fig 1, NOD mice immunized to p277(Val⁶-Val¹¹) in Intralipid developed peptide specific antibodies, while mice immunized to p277(Val⁶-Val¹¹) in PBS showed no antibody responses at all.

### Example 3.

### Antibody isotypes induced by p277(Val⁶-Val¹¹) therapy

The association of p277(Val⁶-Val¹¹) Intralipid therapy with antibodies to p277(Val⁶-Val¹¹) shown in Example 2, suggested that the therapeutic effect might result from a shift in the predominant cytokines produced by the autoimmune T cells. T cells of the CD4 "helper" type have been divided into two groups by the characteristic cytokines they secrete when activated (Mosmann and Coffman, 1989): TH1 cells secrete IL-2, which induces T-cell proliferation, and cytokines such as IFNγ, which mediate tissue inflammation; TH2 cells, in contrast, secrete IL-4, which "helps" B cells produce certain antibody isotypes, and IL-10 and other cytokines, which can "depress" tissue inflammation. The possibility of a shift from TH1 to TH2-like behavior was supported by analysis of the isotypes of the antibodies produced after p277(Val⁶-Val¹¹) therapy.

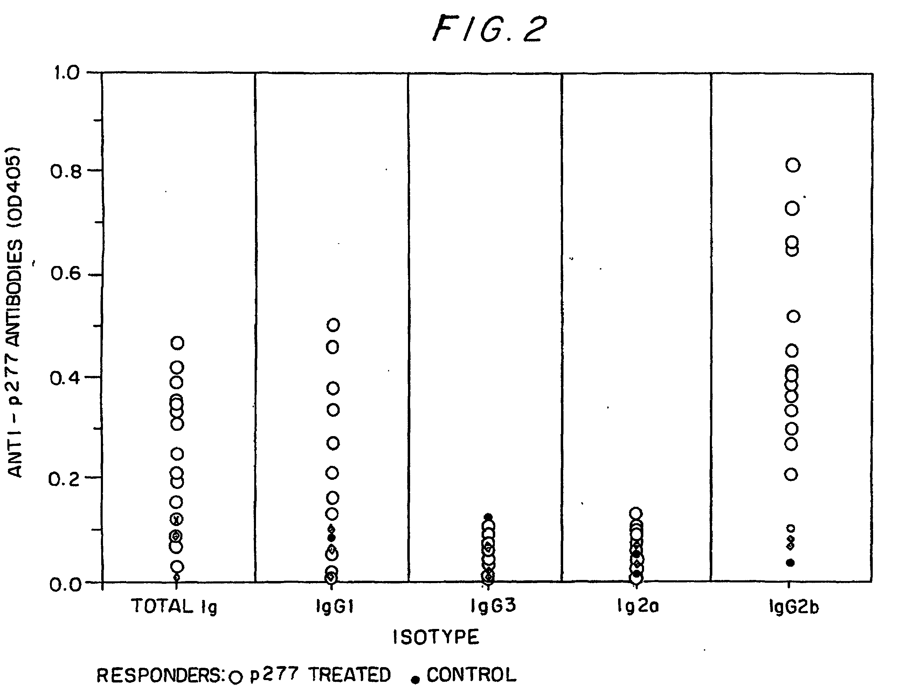

Groups of NOD mice, 3 months old, were treated with p277(Val⁶-Val¹¹) or with PBS in oil as described in Example 2. The sera of individual mice were assayed for the isotypes of their antibodies to p277(Val⁶-Val¹¹) after treatment (12-15 mice per group). The antibody isotypes were detected using an ELISA assay with isotype-specific developing antibody reagents (Southern Biotechnology Associates, Birmingham, AL). The results are shown in Fig. 2, wherein: Antibodies to p277(Val⁶-Val¹¹) in control-treated NOD mice-open circles; in p277(Val⁶-Val¹¹)-treated mice - closed circles. The columns in each experiment show results from equal numbers of mice; an apparent reduction in numbers of circles is caused by superimposition.

Analysis of the antibody isotypes of the anti-p277 antibodies developing after treatment showed them to be exclusively of the IgG1 and IgG2b classes, dependent on TH2 T cells producing IL-4 (Snapper et al., 1993a) and possibly TGFβ (Snapper et al., 1993b). There were no TH1-type IgG2a antibodies induced by p277(Val⁶-Val¹¹) therapy. The development of antibodies to the specific peptide used in treatment is a sign that the autoimmune T-cell responses have shifted from a damaging inflammatory mode called TH1 to a TH2 T-cell response that produces innocuous antibodies and suppresses inflammation and tissue damage (Rabinovitch, 1994).

### Example 4.

### Peptide p277(Val⁶-Val¹¹)/Intralipid therapy induces a specific switch in the cytokine profile

To confirm the idea of a cytokine switch, the cytokines produced by the T cells reactive to the p277(Val⁶-Val¹¹) in the p277(Val⁶-Val¹¹)/Intralipid-treated and control mice were assayed. Concanavalin A (ConA), a T-cell mitogen, was used to activate total splenic T-cells as a control.

Groups of 10 NOD mice, 3 months old, were treated with p277(Val⁶-Val¹¹) in Intralipid (closed bars) or with PBS in Intralipid (open bars; see Example 2). Five weeks later, the spleens of the mice were removed and the spleen cells were pooled. The spleen cells were incubated with Con A or p277(Val⁶-Val¹¹) for 24h (for IL-2 and IL-4 secretion) or for 48h (for IL-10 and IFNγ secretion). The presence of the cytokines in the culture supernatants was quantitated by ELISA, using Pharmingen paired antibodies according to the Pharmingen cytokine ELISA protocol. Pharmingen recombinant mouse cytokines were used as standards for calibration curves. Briefly, flat-bottom 96-well microtiter plates were coated with rat anti-mouse cytokine mAbs for 18h at 4°C, and the culture supernatants or recombinant mouse cytokines were added for 18h at 4°C. The plates were washed, and biotinylated rat anti-mouse cytokine mAbs were added for 45 min at room temperature, then extensively washed, and avidin-alkaline phosphatase was added. The plates were washed, a chromogen substrate (p-nitrophenylphosphate) was added and samples were read at 405nm in an ELISA reader. The results are shown in Fig. 3. The concentrations of cytokines are shown as the OD readings. *P<0.01.

Fig. 3A shows that the spleen cells of control mice secreted both IL-2 and IFNγ upon incubation with p277(Val⁶-Val¹¹). In contrast, the p277(Val⁶-Val¹¹)-treated mice produced significantly less (P<0.01) IL-2 and IFNγ in response to incubation with peptide p277(Val⁶-Val¹¹). This reduction in TH1 cytokines was specific; the p277(Val⁶-Val¹¹) - treated mice maintained their IL-2 and IFNγ cytokine responses to ConA (Fig. 3B). Figs. 3A and 3B show the amounts of IL-10 and IL-4 produced by the spleen cells of the mice. The control mice produced very little IL-4 or IL-10 in response to p277(Val⁶-Val¹¹) or Con A. In contrast, there was a significant increase in IL-10 and IL-4 in response only to p277(Val⁶ -Val¹¹) and only in the p277(Val⁶-Val¹¹)/Intralipid-treated mice (P<0.01). A decrease in IL-2 and IFNγ coupled with an increase in IL-10 and IL-4 confirms the shift from TH1-like behavior to TH2-like behavior. Such a shift might help explain both a decline in T-cell proliferation to p277 shown previously by the inventors (Elias et al., 1991) and the appearance of IgG1 and IgG2b antibodies to p277(Val⁶-Val¹¹) according to the present invention.

### Example 5.

### Spontaneous T-cell proliferative responses to p277(Val⁶-Val¹¹) is reduced by p277(Val⁶-Val¹¹) therapy

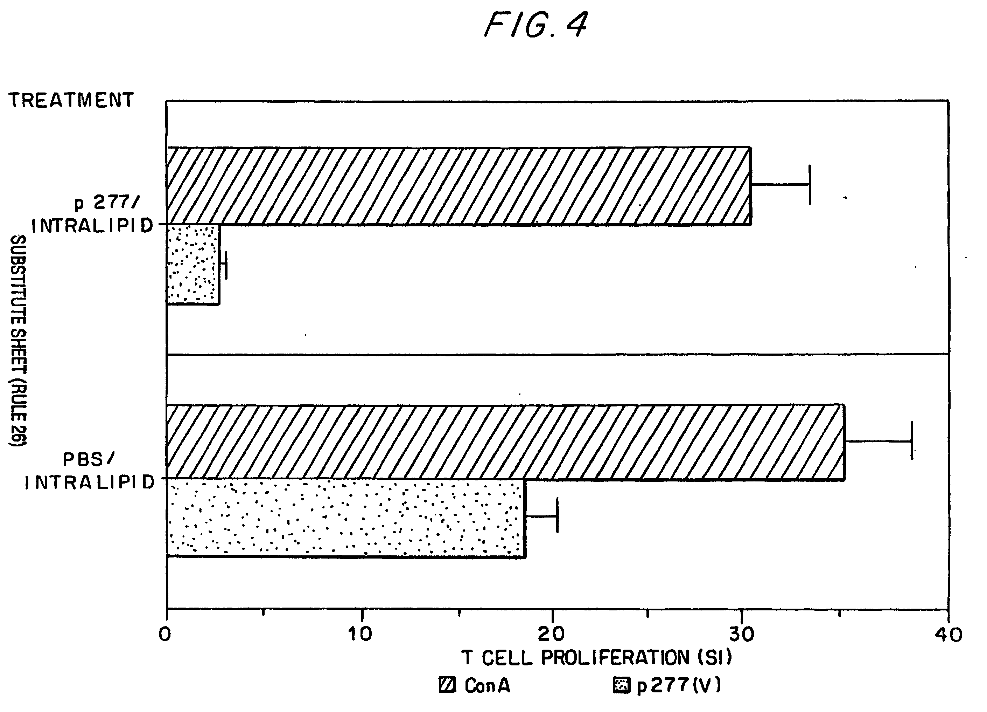

Groups of 5 female mice of the NOD/Lt strain were treated at the age of 3 months with 100 µg of peptide p277(Val⁵-Val¹¹) in Intralipid or with PBS mixed with Intralipid, sc in the back. Five weeks later, the spleens of the mice were removed and the T-cell proliferative responses were assayed in vitro to the T-cell mitogen Con A (1.25 µg/ml) or to p277(Val⁶-Val¹¹) (10 µg/ml) using a standard assay. The results are shown in Fig. 4, wherein: Con A-black striped bars; p277(Val⁶-Val¹¹) - grey bars. The T-cell responses were detected by the incorporation of [³H] thymidine added to the wells in quadruplicate cultures for the last 18 hours of a 3-day culture. The stimulation index (SI) was computed as the ratio of the mean cpm of test cultures to the mean cpm of antigen-containing wells to control wells cultured without antigens or Con A. The standard deviations from the mean cpm were always less than 10%.

As shown in Fig. 4, the control mice tested with PBS/Intralipid showed T-cell proliferative responses to both p277(Val⁶-Val¹¹) and to the T-cell mitogen Con A. In contrast, the mice treated with p277(Val⁶-Val¹¹) in Intralipid showed a decrease in T-cell proliferative reactivity to p277(Val⁶-Val¹¹) but no decrease to Con A. Thus the beneficial effect of p277(Val⁶-Val¹¹) peptide therapy is caused not by inactivating the autoimmune response, but by activating the autoimmunity into a different cytokine mode of behavior (Cohen, 1995). Regulation of destructive autoimmunity is programmed within the immune system (Cohen, 1992); it need only be activated by a suitable signal which requires the peptide together with the lipid vehicle; neither the peptide alone or the lipid without the peptide are effective, as shown in Table 1. These results indicate that metabolizable lipid emulsions may be use defectively as vehicles for therapy of autoimmune diseases. Each disease will require its own specific peptide, but the metabolizable lipid emulsion can be used for the various therapies.

### Example 6.

### Administration of peptide in Intralipid affects development of experimental autoimmune encerhalomyelitis

Experimental autoimmune encephalomyelitis (EAE) is an experimental autoimmune disease of animals that is thought to model aspects of multiple sclerosis (Zamvil and Steinman,1990). EAE can be induced in susceptible strains of rats, such as the Lewis rat, by immunization to myelin basic protein (MBP) in complete Freund's adjuvant (CFA), an emulsion of mineral oil containing killed Mycobacteria. The disease develops about 12 days after immunization and is characterized by paralysis of various degrees due to inflammation of the central nervous system. The paralysis can last up to 6 or 7 days and the rats usually recover unless they die during the peak of their acute paralysis. EAE is caused by T cells that recognize defined determinants of the MBP molecule. The major MBP determinant in the Lewis rat is composed of the peptide sequence 71-90 (Zamvil and Steinman, 1990).

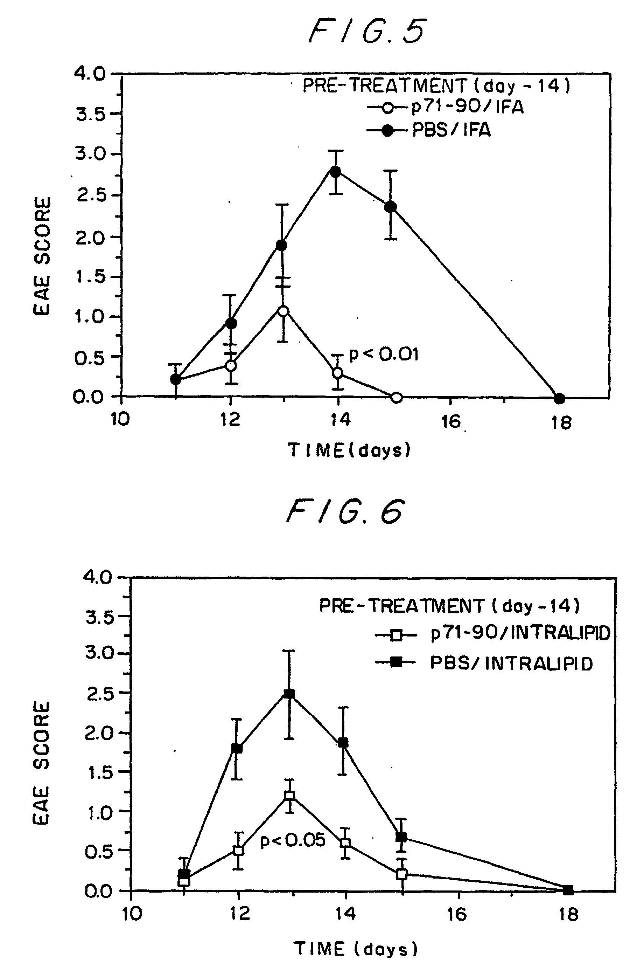

We therefore performed an experiment to test whether administration of the encephalitogenic MBP peptide p71-90 in IFA could also inhibit the development of EAE. Fig. 5 shows that the administration of p71-90 in IFA 14 days before the induction of EAE led to a significant decrease in the maximal degree of paralysis compared to the control treatment with PBS emulsified in IFA, which had no effect on the severity of the disease. Thus, p71-90 given in IFA affects EAE.

However, IFA cannot be administered, as stated above, to humans because it is not metabolizable in the body and causes local inflammation. We therefore treated Lewis rats with p71-90 in Intralipid. Figure 6 shows the results. The rats that had received p71-90 in Intralipid developed significantly less paralysis than did the control rats treated with PBS/Intralipid. Therefore, it can be concluded that a relevant peptide such as p71-90 administered in Intralipid is capable of modulating EAE in rats. Hence, the effects of peptide/Intralipid treatment are not limited to only one peptide, in one species, or to only one autoimmune disease.

### Example 7

### Effectiveness of new vs. aged 10% Intralipid emulsion

10% Intralipid emulsion was used to treat 12 week old NOD female mice with p277(Val⁶-Val¹¹). The emulsion was used either on the day the sealed bottle was opened, or 4 months later, after exposure to atmospheric air. The pH of the emulsion was tested at the time of preparing the peptide+emulsion for treatment. Aging was marked by a fall in pH from 8.2 to 6.7. In each experiment 10 mice were treated with the peptide+emulsion preparation, 10 mice received the emulsion alone, and 10 mice were untreated. The results are shown in Table 3.

**Table 2**

| Group | Treatment | Emulsion PH | Diabetes (%) | Mortality (%) |
|---|---|---|---|---|
| 1 | peptide+emulsion | 8.2 | 20* | 10* |
| 2 | emulsion | -"- | 90 | 70 |
| 3 | peptide+emulsion | 6.7 | 60 | 40 |
| 4 | emulsion | -"- | 80 | 60 |
| 5 | untreated | - | 90 | 80 |

| | | | | |
|---|---|---|---|---|
| * p < 0.01 | | | | |

It can be seen that the placebo-treated mice (emulsion only, groups 2 and 4) and the untreated mice (group 5) developed a similar incidence of diabetes, 80-90% at 6 months of age. In contrast, treating the mice with peptide in the newly opened emulsion protected 80% of the mice from diabetes. However, using the "aged" emulsion only protected 40%. Therefore, the emulsion was chemically unstable after exposure to air, as shown by the marked decrease in pH value. This change is relevant to its biological activity. Hence, the Intralipid is a biologically active carrier whose functional properties depend on the pH and not only on the presence of inert lipid.

### REFERENCES

1. Allegretta, M., Nicklas, J.A., Sriram, S. and Albertini, R.J., *Science* 247:718-721 (1990).
2. Banchereau, J. and Rybak, M.E., in: *The Cytokine Handbook*, 2nd Ed., A, Thompson Ed. Academic Press New York, pp 99 (1994).
3. Cohen, I.R., *Immunology Today* 13:490-494 (1992).
4. Cohen, I.R., in: *Selective Immunosuppression: Basic Concepts and Clinical Applications* (Eds. Adorini, L., Capra, D.J., Waldmann, H.) *Chem. Immunol* Karger, Basel, 60:150-60 (1995).
5. Kristiansen, T., Sparrman, M. and Heller, L., *J. Biosci* 5 (suppl. 1) : 149-155 (1983).
6. Liblau, R.S., Singer, S.M. and McDevitt, H.O., *Immunology Today* 16:34 38 (1995).
7. Moore, K.V., O'Garra, A., de Waal Malefyt, R., Vieira, P. and Mosmann, T.R., *Ann. Rev. Immunol.* 11:165-190 (1993) .
8. Mosmann, T.R. and Coffman R.L., *Ann. Rev Immunol.* 7:145-173 (1989).
9. Ota, K., Matsui, M., Milford, E.L., Mackin, G.A., Weiner, H.I. and Hafler, D.A., *Nature* 346:183-187 (1990).
10. Rabinovitch, A., Sorensen, O., Sua-Pinzon, W.K., Rajotta, R.V. and Bleakley, R.C., *Diabetologia* 37:833-837 (1994).
11. Romagnani, S., *Ann. Rev. Immunol.* 12:227-257 (1994).
12. Snapper, C.M. and Mond, J.J., *Immunology Today* 14:15-17 (1993).
13. Snapper, C.M., Waegell, W., Beernink, H. and Dasch, J.R., *J. Immunol.* 151:4625-36 (1993).
14. Zarnvil, S.S. and Steinman, L., *Ann. Rev. Immunol.* 8:579-621 (1990).

## Claims

1. A therapeutic preparation for treatment of a T cell mediated autoimmune disease or condition comprising an antigen and a biologically active carrier, wherein the antigen is an antigen recognized by inflammatory T cells associated with the pathogenesis of said disease or condition, and wherein the said carrier is a fat emulsion comprising 10-20% triglycerides of plant and/or animal origin, 1,2-2.4% phospholipids of plant and/or animal origin, 2.25-4.5% osmo-regulator, 0-0.05% anti-oxidant, and sterile water to complete 100%.

2. A preparation according to claim 1, wherein the triglycerides are of plant origin, preferably derived from soybean oil, or of animal origin, preferably derived from egg yolk or bovine serum.

3. A preparation according to claim 1 or 2, wherein the phospholipids are of plant origin, preferably derived from soybeans, or of animal origin, preferably derived from egg yolk or bovine serum.

4. A preparation according to any one of claims 1 to 3, wherein the osmo-regulator is selected from the group comprising glycerol, sorbitol and xylitol.

5. A preparation according to any one of claims 1 to 4, comprising 0.05% tocopherol as anti-oxidant.

6. A preparation according to any one of claims 1 to 4, wherein the biologically active carrier is a fat emulsion comprising 10% soybean oil, 1-2% egg-yolk phospholipids, 2.5% glycerol and sterile water to complete 100%.

7. A preparation according to any one of claims 1 to 4, wherein the biologically active carrier is a fat emulsion comprising 20% soybean oil, 2.4% egg-yolk phospholipids, 2.5% glycerol and sterile water to complete 100%.

8. A preparation according to any one of claims 1 to 4, wherein the biologically active carrier is a fat emulsion comprising 5% soybean oil, 5% medium chain triglycerides, 1.2% egg-yolk lecithin, 2.5% glycerol and sterile water to complete 100%.

9. A preparation according to any one of claims 1 to 8 which causes shifting of an individual's T-cell cytokine response from TH1 to TH2 expressed by causing a decrease in IL-2 or IFN-γ-cell cytokine response and an increase in IL-4 or IL-10 T-cell cytokine response.

10. A preparation according to any one of claims 1 to 9 for the treatment of insulin dependent diabetes mellitus (IDDM), wherein said antigen comprises a peptide derived from the human heat shock protein 60 (hsp60) that is recognized by inflammatory T-cells associated with the pathogenesis of IDDM, wherein said peptide is selected from the group of peptides listed in Table 1.

11. A preparation according to claim 10 for the treatment of IDDM, comprising the peptide p277 and a fat emulsion comprising 10% soybean oil, 1.2% egg-yolk phospholipids, 2.5% glycerol and sterile water to complete 100%.

12. A preparation according to claim 11 for the treatment of IDDM, comprising the peptide p277(Val6-Val11) and a carrier consisting of a fat emulsion comprising 10% soybean oil, 1.2% egg-yolk phospholipids, 2.5% glycerol and sterile water to complete 100%.

13. A preparation according to any one of claims 1 to 9 for the treatment of multiple sclerosis, wherein said antigen comprises a peptide derived from myelin basic protein (MBP) that is recognized by T-cells involved in the pathogenesis of multiple sclerosis.

14. Use of a fat emulsion comprising 10-20% triglycerides of plant and/or animal origin, 1.2-2-4% phospholipids of plant and/or animal origin, 2.25-4.5% osmo-regulator, 0-0.5% anti-oxidant, and sterile water to complete 100%, for the manufacture of a therapeutic preparation according to claim 1.

15. Use of a fat emulsion comprising 10% soybean oil, 1.2% egg-yolk phospholipids, 2.5% glycerol, and sterile water to complete 100%, for the manufacture of a therapeutic preparation according to claim 6.

16. A preparation according to any one of claims 1 to 4, wherein the fat emulsion is Intralipid 10%.

17. A preparation according to any one of claims 1 to 4, wherein the fat emulsion is Lipofundin 10%.

18. A preparation according to claim 11 for the treatment of IDDM, comprising the peptide p277 and a fat emulsion, wherein the fat emulsion is Intralipid 10%.

19. A preparation according to claim 12 for the treatment of IDDM, comprising the peptide p277(Val6-Val11) and a fat emulsion, wherein the fat emulsion is selected from Intralipid 10% or Lipofundin 10%.

## Patentansprüche

1. Therapeutisches Präparat zur Behandlung einer/eines T-Zell-vermittelten Autoimmunerkrankung oder -zustands, umfassend ein Antigen und einen biologisch aktiven Träger, wobei das Antigen ein Antigen ist, das durch Entzündung betreffende T-Zellen, die mit der Pathogenese der Erkrankung oder des Zustandes assoziiert sind, erkannt wird, und wobei der Träger eine Fettemulsion ist, enthaltend 10 bis 20% Triglyceride pflanzlichen und/oder tierischen Ursprungs, 1,2-2,4% Phospholipide pflanzlichen und/oder tierischen Ursprungs, 2,25-4,5% Osmo-Regulator, 0,05% Antioxidans, und steriles Wasser ad 100%.

2. Präparat nach Anspruch 1, wobei die Triglyceride pflanzlichen Ursprungs sind, vorzugsweise abgeleitet von Sojabohnenöl, oder tierischen Ursprungs, vorzugsweise abgeleitet von Eidotter oder Rinderserum.

3. Präparat nach Anspruch 1 oder 2, wobei die Phospholipide pflanzlichen Ursprungs sind, vorzugsweise abgeleitet von Sojabohnen, oder tierischen Ursprungs, vorzugsweise abgeleitet von Eidotter oder Rinderserum.

4. Präparat nach einem der Ansprüche 1 bis 3, wobei der Osmo-Regulator ausgewählt ist aus der Gruppe umfassend Glycerin, Sorbit und Xylit.

5. Präparat nach einem der Ansprüche 1 bis 4, umfassend 0,05% Tocopherol als Antioxidans.

6. Präparat nach einem der Ansprüche 1 bis 4, wobei der biologisch aktive Träger eine Fettemulsion ist, umfassend 10% Sojabohnenöl, 1-2% Eidotter-Phospholipide, 2,5% Glycerin und steriles Wasser ad 100%.

7. Präparat nach einem der Ansprüche 1 bis 4, wobei der biologisch aktive Träger eine Fettemulsion ist, umfassend 20% Sojabohnenöl, 2,4% Eidotter-Phospholipide, 2.5% Glycerin und steriles Wasser ad 100%.

8. Präparat nach einem der Ansprüche 1 bis 4, wobei der biologisch aktive Träger eine Fettemulsion ist, umfassend 5% Sojabohnenöl, 5% Triglyceride mittlerer Kettenlänge, 1.2% Eidotter-Lecithin, 2,5% Glycerin und steriles Wasser ad 100%.

9. Präparat nach einem der Ansprüche 1 bis 8, das eine Verschiebung der T-Zell-Cytokinantwort eines Individuums von TH1 to TH2 verursacht, was sich darin äußert, dass eine Abnahme an IL-2- oder IFN-γ-T-Zell-Cytokinantwort und eine Zunahme an IL-4 oder IL-10 T-Zell-Zytokinantwort bewirkt wird.

10. Präparat nach einem der Ansprüche 1 bis 9 zur Behandlung von Insulinabhängigem Diabetes mellitus (IDDM), wobei das Antigen ein Peptid umfasst, das vom menschlichen Hitzeschock-Protein 60 (hsp60) abgeleitet ist, das durch inflammatorische T-Zellen, die mit der Pathogenese von IDDM assoziiert sind, erkannt wird, wobei das Peptid ausgewählt ist aus der in Tabelle 1 aufgelisteten Gruppe von Peptiden.

11. Präparat nach Anspruch 10 zur Behandlung von IDDM, umfassend das Peptid p277 und eine Fettemulsion, umfassend 10% Sojabohnenöl, 1.2% Eidotter-Phospholipide, 2,5% Glycerin und steriles Wasser ad 100%.

12. Präparat nach Anspruch 11 zur Behandlung von IDDM, umfassend das Peptid p277 (Val6-Val11) und einen Träger bestehend aus einer Fettemulsion, umfassend 10% Sojabohnenöl, 1.2% Eidotter-Phospholipide, 2,5% Glycerin und steriles Wasser ad 100%.

13. Präparat nach einem der Ansprüche 1 bis 9 zur Behandlung von Multipler Sklerose, wobei das Antigen ein Peptid umfasst, das vom basischen Myelin Protein (MBP) abgeleitet ist, das durch T-Zellen erkannt wird, die in die Pathogenese von Multipler Sklerose involviert sind.

14. Verwendung einer Fettemulsion, umfassend 10-20% Triglyceride pflanzlichen und/oder tierischen Ursprungs, 1,2-2,4% Phospholipide pflanzlichen und/oder tierischen Ursprungs, 2,25-4,5% Osmo-Regulator, 0-0,5% Antioxidans, und steriles Wasser ad 100%, für die Herstellung eines therapeutischen Präparats nach Anspruch 1.

15. Verwendung einer Fettemulsion, umfassend 10% Sojabohnenöl, 1,2% Eidotter-Phospholipide, 2,5% Glycerin, und steriles Wasser ad 100%, für die Herstellung eines therapeutischen Präparats nach Anspruch 6.

16. Präparat nach einem der Ansprüche 1 bis 4, wobei die Fettemulsion Intralipid 10% ist.

17. Präparat nach einem der Ansprüche 1 bis 4, wobei die Fettemulsion Lipofundin 10% ist.

18. Präparat nach Anspruch 11 zur Behandlung von IDDM, umfassend das Peptid p277 und eine Fettemulsion, wobei die Fettemulsion Intralipid 10% ist.

19. Ein Präparat nach Anspruch 12 zur Behandlung von IDDM, umfassend das Peptid p277 (Val6-Val11) und eine Fettemulsion, wobei die Fettemulsion ausgewählt wird aus Intralipid 10% oder Lipofundin 10%.

## Revendications

1. Préparation thérapeutique pour le traitement de maladies auto-immunes ou d'états médiés par le lymphocyte T, comprenant un antigène et un véhicule actif d'un point de vue biologique, dans laquelle l'antigène est un antigène reconnu par des lymphocytes T inflammatoires associés à la pathogénie de ladite maladie ou dudit état, et dans laquelle ledit véhicule est une émulsion grasse comprenant 10 à 20 % de triglycérides d'origine animale et/ou végétale, 1,2 à 2,4 % de phospholipides d'origine animale et/ou végétale, 2,25 à 4,5 % de régulateur osmotique, 0 à 0,05 % d'anti-oxydant et de l'eau stérile pour compléter à 100 %.

2. Préparation selon la revendication 1, dans laquelle les triglycérides sont d'origine végétale, de préférence, dérivés de l'huile de soja, ou d'origine animale, de préférence dérivés du jaune d'oeuf ou du sérum bovin.

3. Préparation selon la revendication 1 ou 2, dans laquelle les phospholipides sont d'origine végétale, de préférence, dérivés des graines de soja, ou d'origine animale, de préférence, dérivés du jaune d'oeuf ou du sérum bovin.

4. Préparation selon l'une quelconque des revendications de 1 à 3 dans laquelle le régulateur osmotique est sélectionné à partir d'un groupe comprenant du glycérol, du sorbitol et du xylitol.

5. Préparation selon l'une quelconque des revendications de 1 à 4, comprenant 0,05 % de tocophérol comme anti-oxydant.

6. Préparation selon l'une quelconque des revendications de 1 à 4, dans laquelle le véhicule actif d'un point de vue biologique est une émulsion grasse comprenant 10 % d'huile de soja, 1 à 2 % de phospholipides de jaune d'oeuf, 2,5 % de glycérol, et de l'eau stérile pour compléter à 100 %.

7. Préparation selon l'une quelconque des revendications de 1 à 4, dans laquelle le véhicule actif d'un point de vue biologique est une émulsion grasse comprenant 20 % d'huile de soja, 2,4 % de phospholipides de jaune d'oeuf, 2,5 % de glycérol, et de l'eau stérile pour compléter à 100 %.

8. Préparation selon l'une quelconque des revendications de 1 à 4, dans laquelle le véhicule actif d'un point de vue biologique est une émulsion grasse comprenant 5 % d'huile de soja, 5 % de triglycérides à chaîne moyenne, 1,2 % de lécithine de jaune d'oeuf, 2,5 % de glycérol, et de l'eau stérile pour compléter à 100 %.

9. Préparation selon l'une quelconque des revendications de 1 à 8, laquelle provoque le décalage de la réponse en cytokines exprimées par les lymphocytes T d'un individu de TH1 vers TH2, exprimé en générant une diminution de la réponse de la cytokine IFN-γ ou IL-2 des lymphocytes T et une augmentation de la réponse de la cytokine IL-10 ou IL-4 des lymphocytes T.

10. Préparation selon l'une quelconque des revendications 1 à 9 destinée au traitement du diabète de type 1 (DID : diabète insulinodépendant), dans laquelle l'antigène comprend un peptide dérivé de la protéine de choc 60 (hsp60), qui est reconnu par les lymphocytes thermiques inflammatoires associés à la pathogénie du DID, dans laquelle ledit peptide est sélectionné dans le groupe de peptides répertoriés dans le tableau 1.

11. Préparation selon la revendication 10 destinée au traitement du DID, comprenant le peptide p277 et une émulsion grasse comprenant 10 % d'huile de soja, 1,2 % de phospholipides de jaune d'oeuf, 2,5 % de glycérol, et de l'eau stérile permettant d'atteindre les 100 %.

12. Préparation selon la revendication 11 destinée au traitement du DID, comprenant le peptide p277 (Val6-Val11) et un véhicule consistant en une émulsion grasse comprenant 10 % d'huile de soja, 1,2 % de phospholipides de jaune d'oeuf, 2,5 % de glycérol, et de l'eau stérile permettant d'atteindre les 100 %.

13. Préparation selon l'une quelconque des revendications de 1 à 9 destinée au traitement de la sclérose en plaque, dans laquelle ledit antigène comprend un peptide dérivé à partir de la protéine basique de la myéline (MBP), qui est reconnue par les lymphocytes T impliqués dans la pathogénie de la sclérose en plaque.

14. Utilisation d'une émulsion grasse comprenant 10 à 20 % de triglycérides d'origine animale et/ou végétale, 1,2 à 2,4 % de phospholipides d'origine animale et/ou végétale, 2,25 à 4,5 % de régulateur osmotique, 0 à 0,05 % d'anti-oxydant et de l'eau stérile pour compléter à 100 %, pour la fabrication d'une préparation thérapeutique selon la revendication 1.

15. Utilisation d'une émulsion grasse comprenant 10 % d'huile de soja, 1,2 % de phospholipides de jaune d'oeuf, 2,5 % de glycérol, et de l'eau stérile pour compléter à 100 % pour la fabrication d'une préparation thérapeutique selon la revendication 6.

16. Préparation selon l'une quelconque des revendications 1 à 4, dans laquelle l'émulsion grasse est de l'Intralipide 10 %.

17. Préparation selon l'une quelconque des revendications 1 à 4, dans laquelle l'émulsion grasse est de la Lipofundine 10 %.

18. Préparation selon la revendication 11 destinée au traitement de DID, comprenant un peptide p227 et une émulsion grasse, dans laquelle préparation l'émulsion grasse est de l'Intralipide 10 %.

19. Préparation selon la revendication 12 destinée au traitement de DID, comprenant un peptide p227 (Val6-Val11) et une émulsion grasse, dans laquelle l'émulsion grasse est sélectionnée parmi l'Intralipide 10 % ou la Lipofundine 10 %.
